# EUROPEAN PATENT APPLICATION

(11) **EP 4 335 859 A1**
(43) Date of publication of application: **13.03.2024**
(21) Application number: 22798959.7
(22) Date of filing: 06.05.2022
(51) Int. Cl.: C07H 21/04, A61K 31/7125, A61K 48/00, A61P 25/00, A61P 25/28, A61P 43/00, C12N 15/113, C12Q 1/6813

(54) **METHOD FOR DESIGNING OLIGONUCLEOTIDE HAVING REDUCED CENTRAL TOXICITY**

(30) Priority: 06.05.2021 JP 2021078507; 16.09.2021 JP 2021151543
(71) Applicant: Luxna Biotech Co., Ltd., Suita-shi, Osaka 565-0871 (JP)
(72) Inventor: KAWANOBE, Takaaki, Suita-shi, Osaka 565-0871 (JP); YAMAGAMI, Masaki, Suita-shi, Osaka 565-0871 (JP); UMEMOTO, Tadashi, Suita-shi, Osaka 565-0871 (JP)
(74) Representative: Regimbeau
(86) International application number: PCT/JP2022/019561
(87) International publication number: WO 2022/234855

(57) **Abstract**

The present invention provides a method for designing an oligonucleotide having a reduced central toxicity, the method being characterized by including a step for substituting a carbon atom at the 5' position of a sugar portion of at least one nucleoside that constitutes an oligonucleotide having a phosphorothioate modification with a structure represented by formula I (in the formula, R⁶ and R⁷ are each independently a hydrogen atom, a halogen atom or a methyl group) or a structure represented by formula I' (in the formula, R⁴ and R⁵ are each independently a hydrogen atom, a methyl group or an ethyl group (excluding a case where R⁴ and R⁵ are both hydrogen atoms)).

## Description

### [TECHNICAL FIELD]

The present invention relates to a method for designing an oligonucleotide having reduced central toxicity, an oligonucleotide synthesized based on the design results, and a therapeutic agent that comprises the oligonucleotide.

### [BACKGROUND ART]

An antisense oligonucleotide exhibits its activity by hybridizing with a target nucleic acid sequence and suppressing gene expression itself. A variety of artificial nucleic acids have been developed and introduced and their formulations marketed, with the goal of enhancing the nuclease resistance of antisense oligonucleotides and improving binding affinity and specificity for target nucleic acids, but this has led to a new issue in recent years, regarding how to avoid the latent toxicity of artificial nucleic acids.

The toxicity of antisense oligonucleotides can be categorized as either "toxicity due to hybridization with RNA" (off-target toxicity) or "toxicity due to bonding with extracellular and intracellular proteins and metal ions, independently of hybridization with RNA" (non-off-target toxicity). A variety of approaches have been adopted in the attempt to avoid such toxicity.

For example, PTL 1 discloses that non-off-target toxicity can be avoided by appropriate chemical modification of the base or sugar portion of a nucleic acid. PTL 2 discloses that off-target toxicity can be avoided by modification of the carbonyl group at the 2'-position of the base (thymine) and crosslinking the 2'-position and 4'-position of the sugar, to suppress formation of non-Watson-Crick base pairs during hybridization with mRNA. While it is generally known that phosphorothioate modification between nucleosides is a cause of hepatotoxicity (NPL 1, for example), PTL 3 discloses that replacement of phosphorothioate-type nucleic acids with an artificial nucleic acid having a cyclopropane structure at the 5'-position can reduce hepatotoxicity while retaining activity.

Hepatotoxicity is the main indicator for evaluation of toxicity in techniques for reducing toxicity of antisense oligonucleotides, but no detailed knowledge has been obtained in regard to toxicity evaluation in other tissues. For example, while central toxicity is an important evaluation parameter for safety testing of drugs, almost no data has been obtained for central toxicity of antisense oligonucleotides.

In regard to the relationship between antisense oligonucleotides and central toxicity, with particular focus on reducing central toxicity, PTL 4 discloses a correlation between antisense oligonucleic acid sequence and central toxicity (disturbance of intracellular free calcium concentration in neurons). Moreover, to the knowledge of the present inventors, only one publication exists in the literature discussing evaluation of central toxicity by phosphorothioate modification of antisense oligonucleotides (NPL 2). NPL 2 discloses that in a gapmer oligonucleotide with phosphorothioate modification, it is possible to reduce central toxicity by replacing the phosphorothioate bond at the wing section with a phosphodiester bond.

### [CITATION LIST]

### [PATENT LITERATURE]

[PTL 1] International Patent Publication No. WO2018/155450
[PTL 2] International Patent Publication No. WO2018/155451
[PTL 3] International Patent Publication No. WO2020/158910
[PTL 4] International Patent Publication No. 2016/127000

### [NON PATENT LITERATURE]

[NPL 1] Migawa M.T. et al., Nucleic Acids Res, 20; 47(11):5465-5479 (2019)
[NPL 2] Moazami M.P. et al., bioRxiv, doi: https://doi.org/10.1101/2021.02.14.431096 (2021)

### [SUMMARY OF INVENTION]

### [TECHNICAL PROBLEM]

NPL 2 also discloses, however, that with an antisense oligonucleotide (ASO) wherein some of the phosphorothioate modification is removed and a stability-contributing modification is added at the 2'-position of the sugar, the antisense activity can be reduced compared to ASO without removal of the phosphorothioate modification. The reduction in antisense activity is thought to be due to reduced stability of the ASO resulting from removal of the phosphorothioate modification.

It is therefore an object of the present invention to provide a method for designing an ASO whereby reduced central toxicity can be obtained compared to the original ASO, and preferably the antisense activity can also be retained or enhanced, by adding modification at a position other than the 2'-position of the sugar of the ASO, and preferably further removing some of the phosphorothioate modification of the ASO, as well as an ASO synthesized based on the design results.

### [SOLUTION TO PROBLEM]

The present inventors have conducted much research with the goal of solving the problems described above. The target of focus was on modification at the 5'-position of the sugar, rather than modification at the 2'-position or 4'-position of the sugar which is the most widely used method for nucleic acid modification (as in NPL 2, for example). Specifically, it was posited that by introducing a specific structure such as a cyclopropane structure at the 5'-position of the sugar of a phosphorothioate-modified nucleoside, as disclosed in PTL 3, it might be possible to reduce central toxicity by suppression of nonspecific protein binding, while retaining the antisense activity. In PTL 3 it is disclosed that hepatotoxicity is reduced with an ASO having a modification introduced at the 5'-position of the nucleoside sugar, and having the phosphorothioate bond near the modification replaced with a phosphodiester bond. However, since the mechanisms by which hepatotoxicity and central toxicity occur with ASO are thought to be quite different, this postulation is neither described nor suggested in PTL 3 or other prior art documents, and is completely original. The present inventors conducted further research based on this postulation, which led to completion of the present invention.

Specifically, the invention provides the following.
[1] A method for designing an oligonucleotide having reduced central toxicity, comprising:
   (1) a step of replacing the 5'-position carbon atom of the sugar of at least one nucleoside of an oligonucleotide having phosphorothioate modification, with a structure represented by the following formula I: 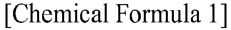 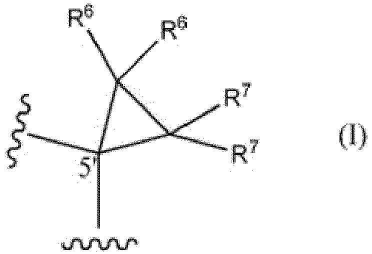 (where R⁶ and R⁷ are each independently a hydrogen atom, halogen atom or methyl group), or the following formula I': 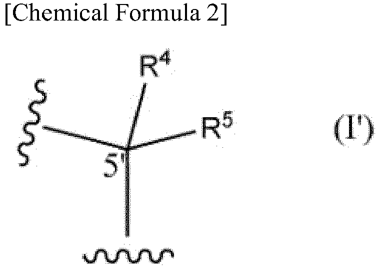 (where R⁴ and R⁵ are each independently a hydrogen atom, methyl group or ethyl group (with the proviso that R⁴ and R⁵ are not both hydrogen atoms)),
      and optionally:
      (2) a step of replacing at least one phosphorothioate bond of the oligonucleotide with a phosphodiester bond.
[2] The method according to [1] above, wherein the oligonucleotide is a gapmer oligonucleotide.
[3] The method according to [2] above, wherein at least one nucleoside with the replacement of step (1) is located in the gap region.
[4] The method according to [2] or [3] above, wherein at least one phosphorothioate bond with the replacement of step (2) is located in the gap region.
[5] The method according to any one of [2] to [4] above, wherein at least one nucleoside composing the 3' wing region and 5' wing region is a crosslinked nucleoside.
[6] A method for evaluating the degree of reduction in central toxicity of an oligonucleotide with phosphorothioate modification, comprising the following steps (1) to (3):
   (1) a step of preparing an oligonucleotide with phosphorothioate modification,
   (2) a step of preparing an oligonucleotide designed by the method according to any one of [1] to [5] above, and
   (3) a step of comparing the central toxicity of the oligonucleotide prepared in step (2) with the central toxicity of the oligonucleotide prepared in step (1).
[7] An oligonucleotide with a phosphorothioate bond having reduced central toxicity, wherein the 5'-position carbon atom of the sugar of at least one nucleoside of the oligonucleotide forms a structure represented by the following formula I:

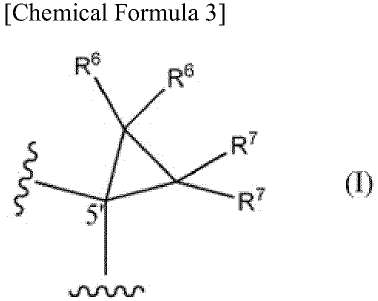

   (where R⁶ and R⁷ are each independently a hydrogen atom, halogen atom or methyl group), or the following formula I':

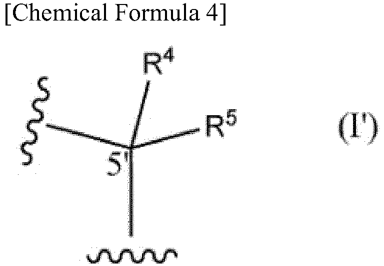

   (where R⁴ and R⁵ are each independently a hydrogen atom, methyl group or ethyl group (with the proviso that R⁴ and R⁵ are not both hydrogen atoms)),
   and optionally at least one internucleoside bond is a phosphodiester bond.
[8] The oligonucleotide according to [7] above, which is a gapmer oligonucleotide.
[9] The oligonucleotide according to [8] above, wherein at least one nucleoside having the structure represented by formula I or I' is located in the gap region.
[10] The oligonucleotide according to [8] or [9] above, wherein at least one phosphodiester bond is located in the gap region.
[11] The oligonucleotide according to any one of [8] to [10] above, wherein at least one nucleoside composing the 3' wing region and 5' wing region is a crosslinked nucleoside.
[12] A reagent for regulating expression of a gene, comprising an oligonucleotide according to any one of [7] to [11] above.
[13] An agent for treating a disease, comprising an oligonucleotide according to any one of [7] to [11] above.
[14] A method for producing an oligonucleotide with low central toxicity, comprising:
   (1) a step of designing an oligonucleotide by the method according to any one of [1] to [5] above, and
   (2) a step of synthesizing the oligonucleotide designed in step (1).
[15] A method for regulating expression of a gene, comprising administering an oligonucleotide according to any one of [7] to [11] above.
[16] A method for treating a disease in mammals, comprising administering an effective amount of an oligonucleotide according to any one of [7] to [11] above to a mammal.
[17] An oligonucleotide according to any one of [7] to [11] above, which is for use in treatment of a disease.
[18] The use of an oligonucleotide according to any one of [7] to [11] above, for production of an agent for treating a disease.

### [ADVANTAGEOUS EFFECTS OF INVENTION]

According to the invention it is possible to design and produce a highly safe antisense oligonucleotide having reduced central toxicity, by replacing a phosphorothioate-modified nucleotide, which is used in numerous developed antisense products, with a sugar 5'-position modified nucleotide.

### [BRIEF DESCRIPTION OF DRAWINGS]

[Fig. 1]

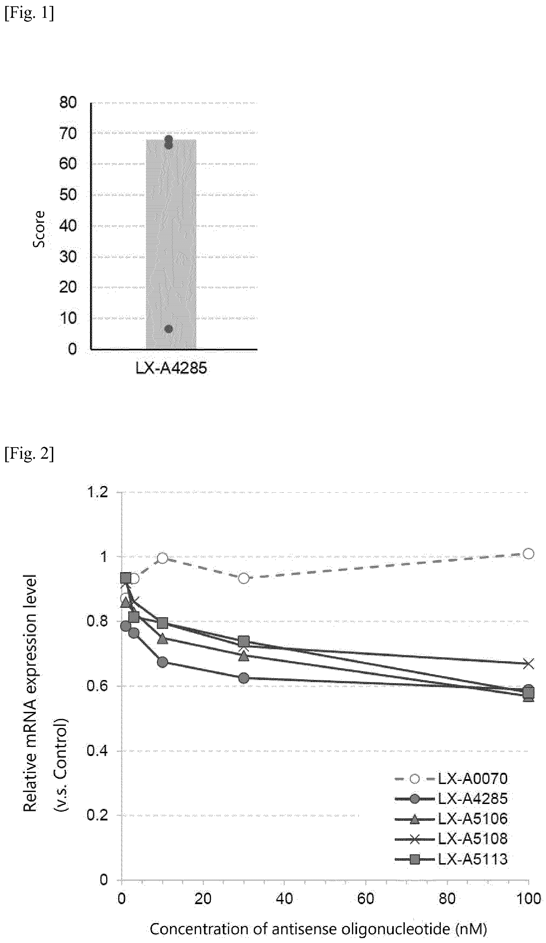

   Fig. 1 shows measurement results for the behavior score after intracerebroventricular administration of an antisense oligonucleotide (LX-A4285) to mice.
[Fig. 2]
   Fig. 2 shows evaluation results for antisense effect, after administration of antisense oligonucleotides (LX-A0070, LX-A4285, LX-A5106, LX-A5108 and LX-A5113) to human neuroblasts in mice.
[Fig. 3]

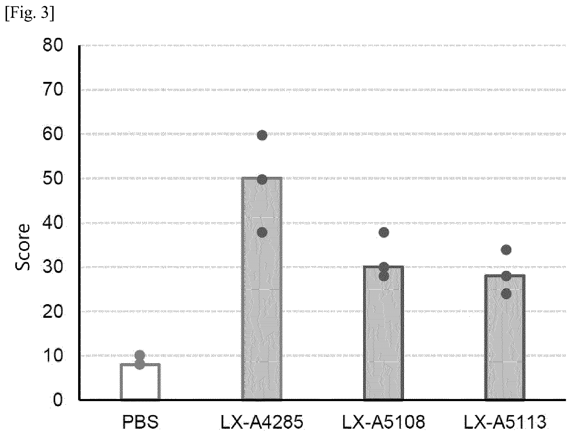

   Fig. 3 shows measurement results for the behavior score after intracerebroventricular administration of antisense oligonucleotides (LX-A4285, LX-A5108 and LX-A5113) to mice.

### [DESCRIPTION OF EMBODIMENTS]

### 1. Method for designing oligonucleotide having reduced central toxicity

The present invention has been completed upon finding that it is possible to design an oligonucleotide having reduced central toxicity, by using a nucleoside having a 5'-cyclopropane structure as a modified nucleotide to replace a phosphorothioate-modified nucleotide.

Specifically, the present invention provides a method for designing an oligonucleotide having reduced central toxicity (hereunder also referred to as "design method of the invention"), comprising:
(1) a step of replacing the 5'-position carbon atom of the sugar of at least one nucleoside of an oligonucleotide having phosphorothioate modification (hereunder also referred to as "PS modified nucleotide"), with a structure represented by the following formula I:

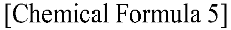

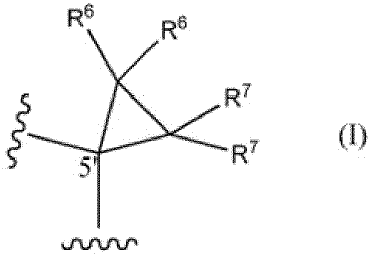

   (where R⁶ and R⁷ are each independently a hydrogen atom, halogen atom or methyl group), or the following formula I':

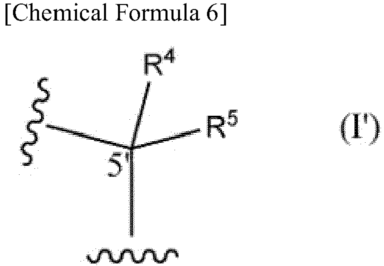

   (where R⁴ and R⁵ are each independently a hydrogen atom, methyl group or ethyl group (with the proviso that R⁴ and R⁵ are not both hydrogen atoms)). According to one aspect, R⁶ and R⁷ are both hydrogen atoms. As used herein, the term "oligonucleotide" also includes pharmacologically acceptable salts of oligonucleotides.

From the viewpoint of further reducing central toxicity, the design method of the invention may also comprise (2) a step of replacing at least one phosphorothioate bond of the oligonucleotide with a phosphodiester bond.

According to another aspect, the invention provides a method for reducing central toxicity of an oligonucleotide (hereunder also referred to as "toxicity reducing method of the invention"), comprising:
(1') a step of replacing the 5'-position carbon atom of the sugar of at least one nucleoside of a PS modified nucleotide with a structure represented by the following formula I:

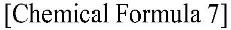

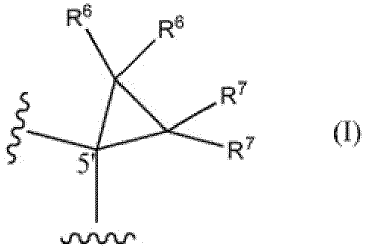

(where R⁶ and R⁷ are each independently a hydrogen atom, halogen atom or methyl group), or the following formula I':

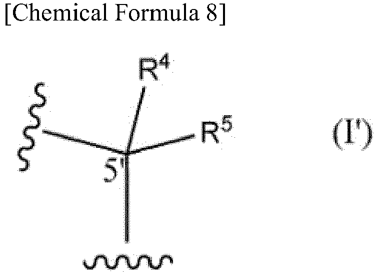

(where R⁴ and R⁵ are each independently a hydrogen atom, methyl group or ethyl group (with the proviso that R⁴ and R⁵ are not both hydrogen atoms)). According to one aspect, R⁶ and R⁷ are both hydrogen atoms.

The toxicity reducing method of the invention may also comprise (2') a step of replacing at least one phosphorothioate bond of the oligonucleotide with a phosphodiester bond.

For aspects applying to both the design method of the invention and the toxicity reducing method of the invention, both methods may be referred to collectively as "methods of the invention".

According to the invention, an "oligonucleotide having phosphorothioate modification" is an oligonucleotide wherein at least one internucleoside bond of the oligonucleotide is a phosphorothioate bond (that is, an oxygen atom of the phosphoric acid group between the nucleosides is replaced with a sulfur atom). Phosphorothioate modification is commonly employed to increase *in vivo* stability, and especially enzyme resistance, of the naturally-occurring oligonucleotides DNA or RNA. Such phosphorothioate-modified oligonucleotides have a common problem in that they are toxic.

The methods of the invention were completed as a result of further research focused on central toxicity, as one of the toxicities of phosphorothioate-modified oligonucleotides, with the goal of reducing it.

The term "central toxicity", or "central nervous system toxicity", for the purpose of the invention, refers to toxicity findings in the central nervous system, identified by general changes in condition or histopathological changes in the brain, of an animal in the range of rodents to non-human primates or humans. In rodents, for example, common changes of state may include minor reversible symptoms such as irritability, reduced spontaneous movement, slow respiration or blepharoptosis, and serious symptoms such as convulsion and death. The "central toxicity" for the invention in particular can be evaluated by a behavior score according to the Irwin test (Irwin S., Psychopharmacologia. 1968; 13(3): 222-257).

According to the invention, "reduced central toxicity" means that the central toxicity of an oligonucleotide after having carried out step (1) or (1') of the methods of the invention (that is, a designed oligonucleotide or an oligonucleotide with reduced toxicity) (this may hereunder also be referred to as the "invention-treated oligonucleotide") is low, or expected to be low, compared to the central toxicity of the PS modified nucleotide that is the target (for design) of the methods of the invention (this may hereunder also be referred to as "target oligonucleotide"). Specifically, as described under "Examples" below, when the difference between the median for the behavior score for a group administered a target oligonucleotide and the median for the behavior score for a group administered an invention-treated oligonucleotide in an Irwin test is 1 or greater (e.g.: 1, 5, 10, 20 or greater), the oligonucleotide may be evaluated as having reduced central toxicity, and the degree of central toxicity also being evaluated based on the value.

In step (1) of the design method of the invention, it is not necessary to actually synthesize the oligonucleotide, as imagining it is sufficient. Typically, however, the imagined oligonucleotide will be realized in a program that runs on an electronic computer (for example, oligonucleotide designing software, graphic design tools or office software), or on paper. Therefore, the process of designing oligonucleotides expected to have reduced central toxicity and organizing the oligonucleotides in table form, as described in Example 1 below, for example, also qualifies as carrying out the design method of the invention.

The target oligonucleotide may be one that suppresses the function (such as post-transcriptional modification or translation) of target mRNA (as used herein, "mRNA" includes pre-mRNA as well) which includes a sequence that is complementary to the sequence of the oligonucleotide (this may also be referred to hereunder as "supression type ASO"), or it may be one that enhances such a function (this may also be referred to hereunder as "enhancement type ASO"). Supression type ASO will typically form a double-stranded region with the target mRNA, the double-stranded region being cleaved by ribonuclease H (RNase H) to suppress the function of the target mRNA. Enhancement type ASO, on the other hand, will typically form a double-stranded region with the splicing promoting sequence of pre-mRNA, masking the sequence and causing the splicing to be skipped, so that the function of the mRNA is enhanced (restored) (whereby the abundance of the functional protein in a cell increases). Alternatively, it may form a double-stranded region with a binding sequence for an enzyme that degrades the mRNA, which is present in the target mRNA, thus masking the sequence and suppressing degradation of the mRNA, and allowing the function of the mRNA to be enhanced.

The supression type ASO is preferably a gapmer oligonucleotide, from the viewpoint of stably and efficiently cleaving mRNA in the body. According to the invention, a "gapmer oligonucleotide" is a chimeric antisense oligonucleotide having an internal region (also referred to herein as "gap region") having one or more (e.g.: 5, 6, 7, 8, 9, 10, 11, 12, 13, 14, 15 or more) nucleotides that are recognized by RNase H, situated between external regions having one or more (e.g.: 1, 2, 3, 4, 5, 6, 7, 8, 9, 10 or more) nucleotides, modified so as to confer resistance to ribonucleases (throughout the present specification, the 3'-end external region will be referred to as the "3' wing region", and the 5'-end external region will be referred to as the "5' wing region"). At least one nucleoside composing each region, the 3' wing region and 5' wing region, is preferably a crosslinked nucleoside.

From the viewpoint of *in vivo* stability, the enhancement type ASO may have at least one nucleoside modified so as to confer resistance to RNase. The ASO may have all of its nucleoside residues modified, or it may have only some of its nucleoside residues modified (that is, it may be a mixmer oligonucleotide). The modified nucleosides are preferably crosslinked nucleosides.

Examples of nucleosides to form the target oligonucleotide include nucleosides represented by the following formula II.

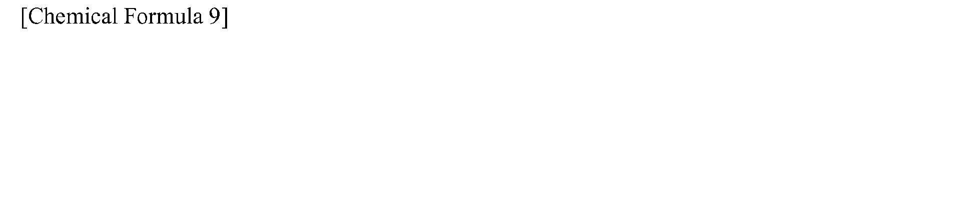

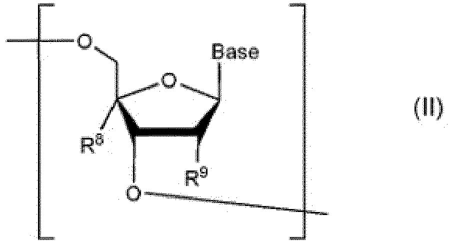

(where:
"Base" represents a purin-9-yl group or 2-oxo-1,2-dihydropyrimidin-1-yl group optionally having one or more arbitrary substituents selected from among an α group, the α group consisting of hydroxyl groups, hydroxyl groups protected with a protecting group for nucleic acid synthesis, straight-chain alkyl groups of 1 to 6 carbon atoms, straight-chain alkoxy groups of 1 to 6 carbon atoms, mercapto groups, mercapto groups protected with a nucleic acid synthesis protecting group, straight-chain alkylthio groups of 1 to 6 carbon atoms, amino groups, straight-chain alkylamino groups of 1 to 6 carbon atoms, amino groups protected with a nucleic acid synthesis protecting group, and halogen atoms, and
R⁸ is a hydrogen atom and R⁹ is a hydrogen atom, a halogen atom or a straight-chain alkoxy group of 1 to 6 carbon atoms optionally substituted with a straight-chain alkoxy group of 1 to 6 carbon atoms.)

Alternatively, R⁸ and R⁹ may together form any of the following chemical structures each representing a divalent group:

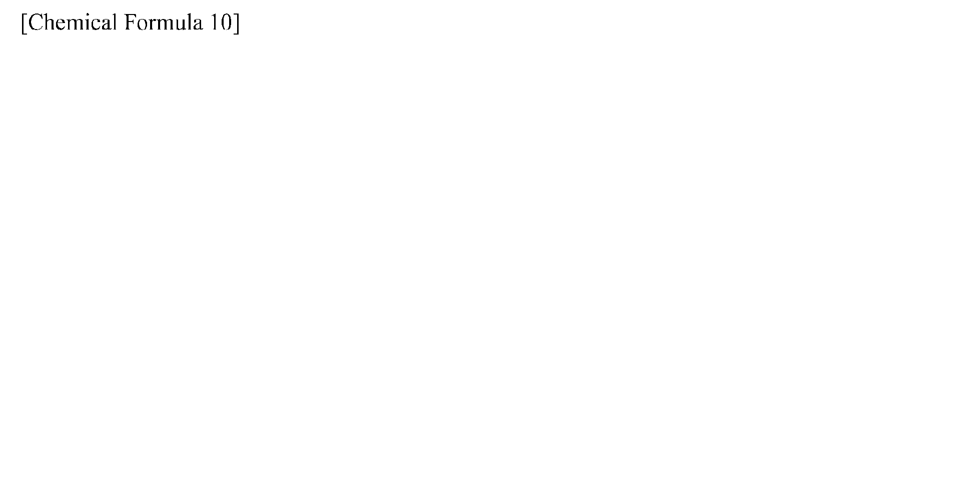

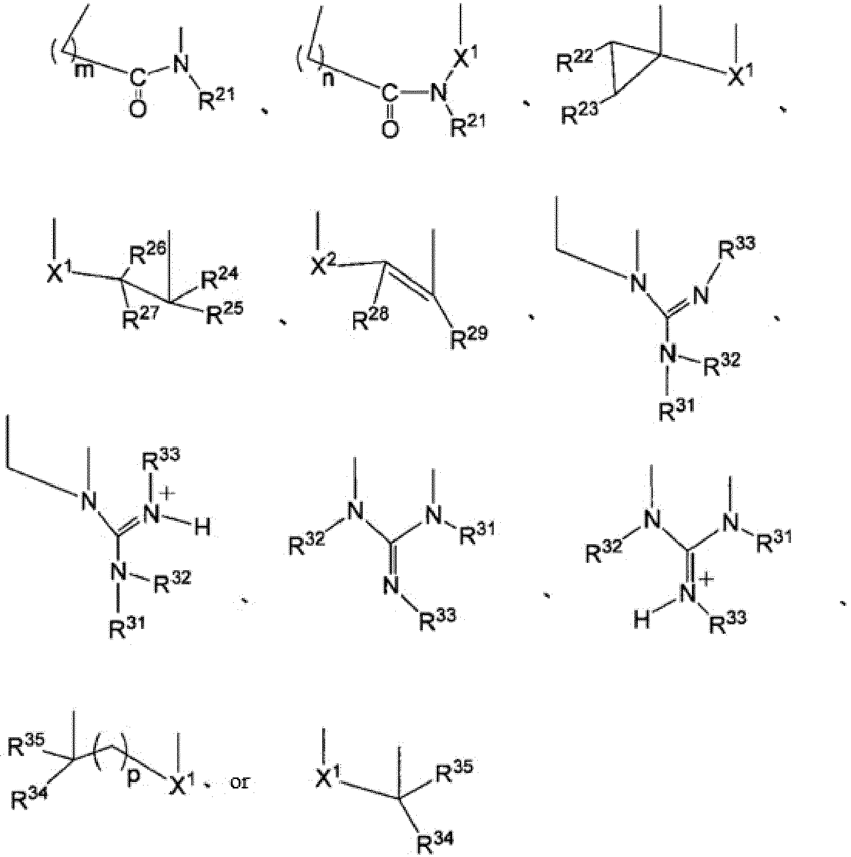

(where:
R²¹ is a hydrogen atom, an optionally branched or cyclic alkyl group of 1 to 6 carbon atoms, an optionally branched or cyclic alkenyl group of 2 to 6 carbon atoms, an aryl group of 3 to 10 carbon atoms optionally having one or more arbitrary substituents selected from among the aforementioned α group and optionally including a heteroatom, an aralkyl group with an aryl group portion of 3 to 12 carbon atoms, optionally having one or more arbitrary substituents selected from among the aforementioned α group and optionally including a heteroatom, or an amino group protecting group for nucleic acid synthesis;
R²² and R²³ are each independently a hydrogen atom; an optionally branched or cyclic alkyl group of 1 to 6 carbon atoms optionally substituted with an aryl group of 3 to 12 carbon atoms optionally including a heteroatom; or an aralkyl group having an aryl group portion of 3 to 12 carbon atoms optionally including a heteroatom; or
R²² and R²³ together represent -(CH₂)_{q}- [where q is an integer of 2 to 5];
R²⁴ and R²⁵ are each independently a group selected from the group consisting of a hydrogen atom; a hydroxyl group; optionally branched or cyclic alkyl groups of 1 to 6 carbon atoms; optionally branched or cyclic alkoxy groups of 1 to 6 carbon atoms; an amino group; or amino groups protected with a nucleic acid synthesis protecting group, or
R²⁴ and R²⁵ together form =C(R³⁶)R³⁷ [where R¹⁶ and R³⁷ each independently represent a hydrogen atom, a hydroxyl group, a hydroxyl group protected with a nucleic acid synthesis protecting group, a mercapto group, a mercapto group protected with a nucleic acid synthesis protecting group, an amino group, a straight-chain or branched alkoxy group of 1 to 6 carbon atoms, a straight-chain or branched alkylthio group of 1 to 6 carbon atoms, a cyanoalkoxy group of 1 to 6 carbon atoms or a straight-chain or branched alkylamino group of 1 to 6 carbon atoms];
R²⁶ and R²⁷ are each independently a hydrogen atom, an optionally branched or cyclic alkyl group of 1 to 6 carbon atoms, an optionally branched or cyclic alkoxy group of 1 to 6 carbon atoms or a straight-chain or branched alkylthio group of 1 to 6 carbon atoms;
R²⁸ is a hydrogen atom, an optionally branched or cyclic alkyl group of 1 to 6 carbon atoms, an optionally branched or cyclic alkoxy group of 1 to 6 carbon atoms or a straight-chain or branched alkylthio group of 1 to 6 carbon atoms;
R²⁹ is a hydrogen atom, a hydroxyl group, an optionally branched or cyclic alkyl group of 1 to 6 carbon atoms, an optionally branched or cyclic alkoxy group of 1 to 6 carbon atoms, an amino group or an amino group protected with a nucleic acid synthesis protecting group;
R³¹, R³² and R³³ are each independently a hydrogen atom, an optionally branched or cyclic alkyl group of 1 to 6 carbon atoms or an amino group protecting group for nucleic acid synthesis;
R³⁴ and R³⁵ are each independently a hydrogen atom, a hydroxyl group, an optionally branched or cyclic alkyl group of 1 to 6 carbon atoms, an optionally branched or cyclic alkoxy group of 1 to 6 carbon atoms, an amino group or an amino group protected with a nucleic acid synthesis protecting group;
m is an integer of 0 to 2;
n is an integer of 0 to 1;
p is an integer of 0 to 1;
X¹ is an oxygen atom, sulfur atom or amino group; and
X² is an oxygen atom or sulfur atom),
and the nucleoside having the structure may be referred to as a "crosslinked nucleoside".

More specifically, examples for the crosslinked nucleoside include nucleosides with the following structural formulas.

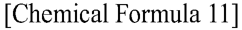

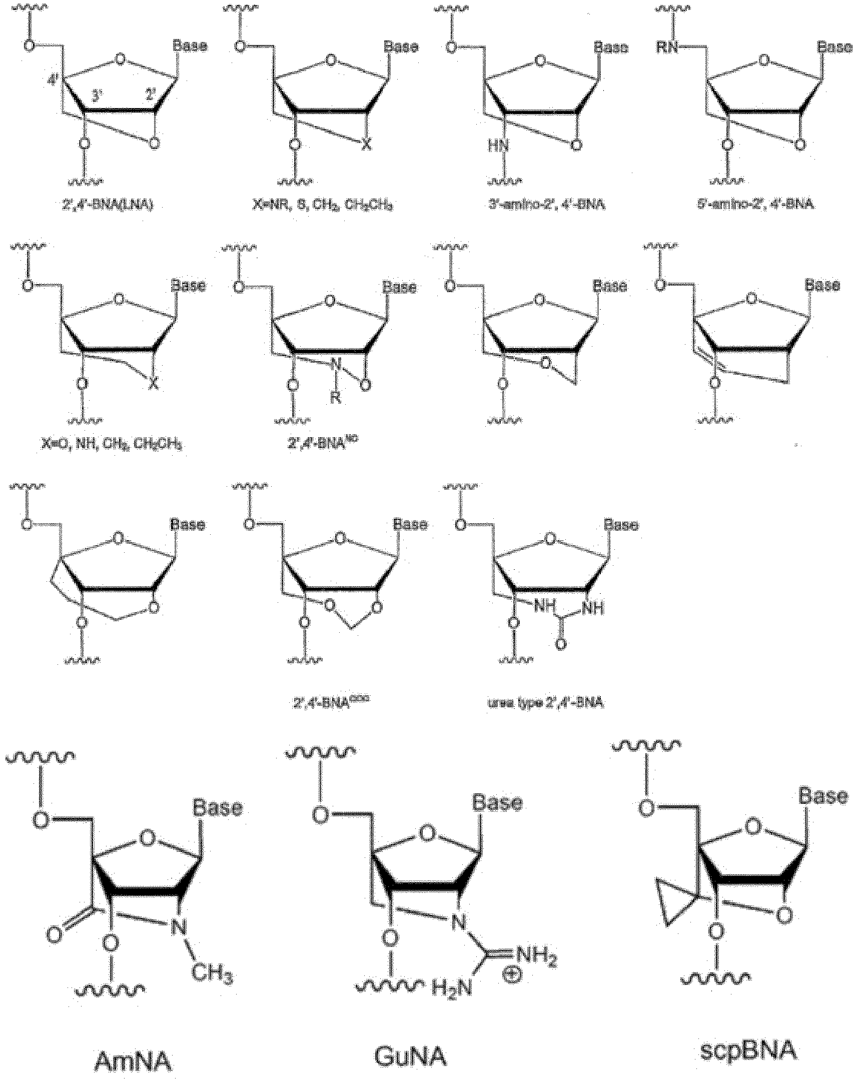

(In the formulas, R represents a hydrogen atom, an optionally branched or cyclic alkyl group of 1 to 7 carbon atoms, an optionally branched or cyclic alkenyl group of 2 to 7 carbon atoms, an aryl group of 3 to 12 carbon atoms optionally including a heteroatom, an aralkyl with an aryl group portion of 3 to 12 carbon atoms optionally including a heteroatom, or an amino group protecting group for nucleic acid synthesis. Preferably, R is a hydrogen atom or a methyl, ethyl, *n*-propyl, isopropyl, phenyl or benzyl group, and more preferably R is a hydrogen atom or a methyl group. The definition of "Base" is the same definition as for formula II.)

The nucleosides composing the target oligonucleotide may be naturally-occurring nucleosides, such naturally-occurring nucleosides including adenosine, N6-methyladenosine, guanosine, uridine, 5-methyluridine, cytidine, deoxyadenosine, N6-methyl-2'-deoxyadenosine, deoxyguanosine, thymidine, deoxyuridine, deoxycytidine and 5-methyl-2'-deoxycytidine.

The length of the target oligonucleotide is not particularly restricted so long as it has antisense activity, but it will typically be 10 to 50 nucleotides in length, preferably 10 to 30 nucleotides in length, more preferably 13 to 30 nucleotides in length and even more preferably 15 to 20 nucleotides in length.

The one or more nucleosides composing the invention-treated oligonucleotide can be represented by the following formula III or III'.

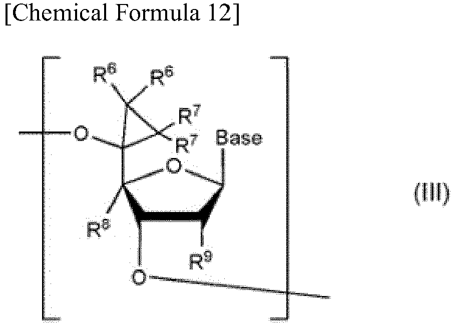

(In the formulas, the definitions of R⁶, R⁷, R⁸, R⁹ and Base are the same definitions as in formula I and formula II. According to one aspect, R⁶ and R⁷ are both hydrogen atoms.)

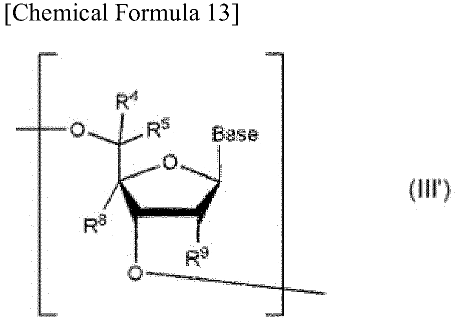

(In the formulas, the definitions of R⁴, R⁵, R⁸, R⁹ and Base are the same definitions as in formula I' and formula II.

As the result of step (1) or (1') of the methods of the invention, the nucleoside having the structure represented by formula I or formula I' may have the target PS modified nucleotide present at any location, but preferably one or more (e.g.: 1, 2, 3, 4, 5, 6, 7, 8, 9, 10 or more) nucleosides are located in the gap region.

As the result of step (2) or (2') of the methods of the invention, the phosphorothioate bond to be replaced may be present between any nucleosides of the target PS modified nucleotide, but preferably one or more (e.g.: 1, 2, 3, 4, 5, 6, 7, 8, 9, 10 or more) phosphorothioate bonds between nucleosides are located in the gap region. In other words, in step (2) or (2') of the methods of the invention it is preferred to replace phosphorothioate bonds located in the gap region of the target PS modified nucleotide with phosphodiester bonds.

As the result of step (2) or (2') of the methods of the invention, the phosphorothioate bond to be replaced may be a bond adjacent at either the 5'-end or 3'-end of the nucleoside replaced by step (1) or (1') in the methods of the invention, or it may be a non-adjacent bond. That is, the nucleotide residue of step (1) or (1') and step (2) or (2') of the invention may each be arbitrarily selected.

In the methods of the invention, further modification may also be made in addition to replacement of 5'-position carbon atoms of sugars of nucleosides, and replacement of phosphorothioate bonds. Such modification may include, but is not limited to, 2'-O-methoxyethyl modification of the sugar, 2'-O-methyl modification of the sugar, 2'-fluoro modification of the sugar, crosslinking of the 2'-position and 4'-position of the sugar (replacement with a crosslinked nucleoside), replacement from a crosslinked nucleoside to a different crosslinked nucleotide, methylation or acetylation of the Base, or replacement of the Base. For replacement of the Base, the replacement is preferably such that it forms a base pair with the target nucleotide of the Base before replacement (that is, so that it forms a base pair with the Base). The base pair is not limited to being a Watson-Crick base pair, and may alternatively be a Hoogsteen base pair or wobble base pair. Examples of crosslinked nucleosides include nucleosides with the following structural formulas.

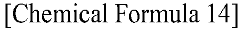

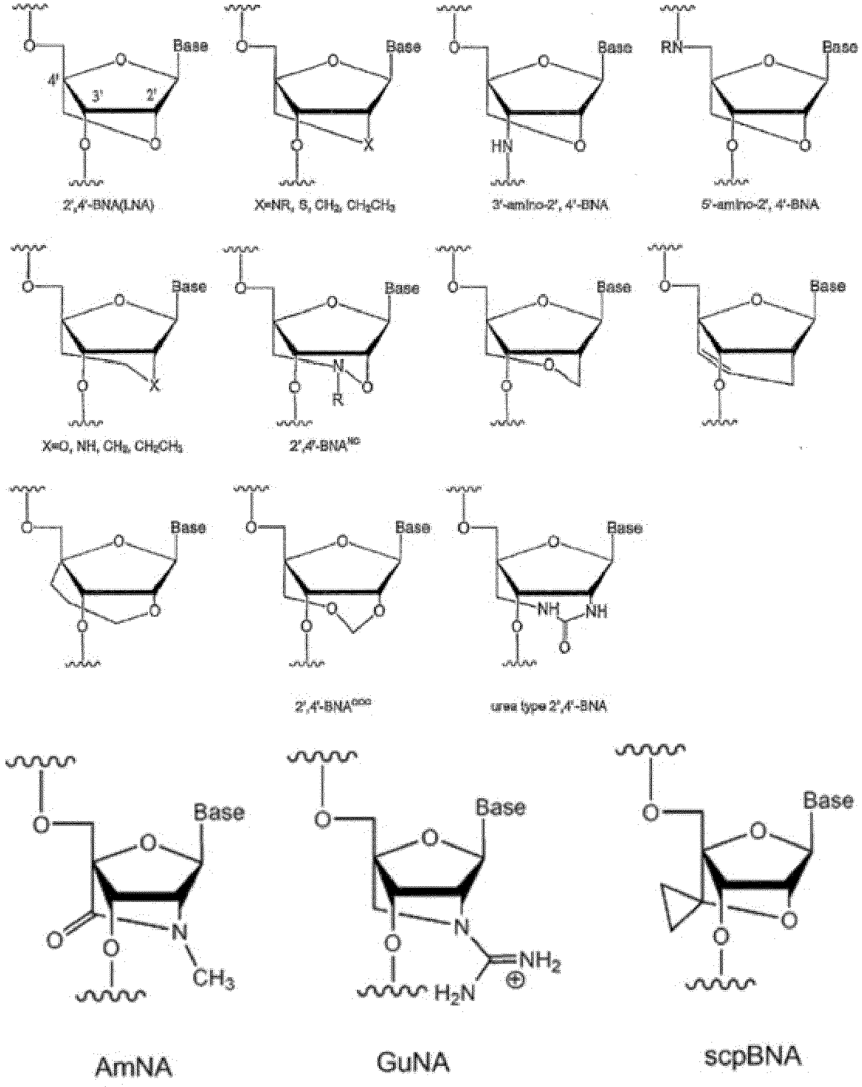

(In the formulas, R represents a hydrogen atom, an optionally branched or cyclic alkyl group of 1 to 7 carbon atoms, an optionally branched or cyclic alkenyl group of 2 to 7 carbon atoms, an aryl group of 3 to 12 carbon atoms optionally including a heteroatom, an aralkyl with an aryl group portion of 3 to 12 carbon atoms optionally including a heteroatom, or an amino group protecting group for nucleic acid synthesis. Preferably, R is a hydrogen atom or a methyl, ethyl, *n*-propyl, isopropyl, phenyl or benzyl group, and more preferably R is a hydrogen atom or a methyl group. The definition of "Base" is the same definition as for formula II.)

By carrying out step (1) or (1') of the methods of the invention for a PS modified nucleotide, it is possible to theoretically design an oligonucleotide having reduced central toxicity compared to the PS modified nucleotide, or to reduce the central toxicity of the PS modified nucleotide. However, it may also be actually confirmed that the invention-treated oligonucleotide has reduced central toxicity, and the degree of reduction in neurotoxicity of the invention-treated oligonucleotide may be actually evaluated.

Yet another aspect of the invention, therefore, provides a method for evaluating the degree of reduction in central toxicity of a PS-modified nucleoside, comprising the following steps (I) to (III):
(I) a step of preparing a PS modified nucleotide,
(II) a step of preparing an oligonucleotide designed by the design method of the invention, and
(III) a step of comparing the central toxicity of the oligonucleotide prepared in step (II) and the central toxicity of the oligonucleotide prepared in step (I) (this method may also be referred to hereunder as "evaluation method of the invention").

The PS modified nucleotide prepared in step (I) of the evaluation method of the invention can be obtained by purchasing a commercial product, by producing it using a publicly known synthesis method, or by delegating its synthesis to a third party. The oligonucleotide prepared in step (II) of the evaluation method of the invention can be obtained by preparation using a publicly known synthesis method as described below based on an oligonucleotide designed by the design method of the invention, or by delegating its synthesis to a third party.

Step (II) of the evaluation method of the invention can be carried out using a method of calculating a score in an Irwin test, or another publicly known method. Step (III) of the evaluation method of the invention can be carried out by comparing calculated scores.

An oligonucleotide designed by the methods of the invention not only has reduced central toxicity, but can also retain antisense activity. According to the invention, "retain antisense activity" means that the antisense activity of the invention-treated oligonucleotide is retained, or higher, compared to the antisense activity of the target oligonucleotide. For supression type ASO, the antisense activity can be evaluated, for example, by contacting each antisense oligonucleotide in an equal amount with SH-SY5Y cells, recovering the mRNA from the cells after at least 24 hours, and comparing the amounts of mRNA, as described in the Examples below. When no statistically significant difference in target mRNA amount is found in the group contacted with the invention-treated oligonucleotide compared to the group contacted with the target oligonucleotide, or if the amount is lower, it may be evaluated that the antisense activity is retained. For enhancement type ASO, on the other hand, an equal amount of the antisense oligonucleotide may be contacted with SH-SY5Y cells, recovering the target mRNA-encoded protein from the cells after at least 24 hours and evaluating the amount of functional protein out of the total protein.

The methods of the invention may therefore further comprise a step of evaluating the antisense activity of the invention-treated oligonucleotide. This step may employ a method of measuring the target mRNA or protein level encoded by the mRNA, or another publicly known method.

### 2. Method for producing oligonucleotide with low central toxicity

According to yet another aspect, the invention provides a method for producing an oligonucleotide with low central toxicity, the method including:
(1) a step of designing an oligonucleotide by the design method of the invention, and
(2) a step of synthesizing the oligonucleotide designed in step (1)
(this method may also be referred to hereunder as "production method of the invention").

According to the invention, "oligonucleotide with low toxicity" means an oligonucleotide having, or expected to have, lower central toxicity than the target oligonucleotide. Specifically, as described under "Examples" below, when the difference between the median for the behavior score for a group administered a target oligonucleotide and the median for the behavior score for a group administered an invention-treated oligonucleotide in an Irwin test is 1 or greater (e.g.: 1, 5, 10, 20 or greater), the oligonucleotide may be evaluated as being an oligonucleotide with low central toxicity, and the degree of central toxicity also being evaluated based on the value.

The method of synthesizing the oligonucleotide in step (2) of the production method of the invention is not particularly restricted, and any publicly known method may be used. Examples of publicly known methods include chemical synthesis methods such as the phosphoroamidite method and H-phosphonate method. A chemical synthesis method may employ a commercially available automatic nucleic acid synthesizer. An amidite is commonly used in such chemical synthesis methods. The amidite is not particularly restricted and may be produced by a publicly known method (such as the method described in PTL 3, International Patent Publication No. WO2020/166551), or alternatively a commercially available amidite product may be used.

The central toxicity of an oligonucleotide produced by the production method of the invention may also be actually evaluated by carrying out the same step as step (III) of the evaluation method of the invention.

### 3. Oligonucleotide with reduced central toxicity

As mentioned above, an oligonucleotide with reduced central toxicity can be designed or produced by carrying out the present invention. According to another aspect, therefore, the invention provides an oligonucleotide with a phosphorothioate bond, having reduced central toxicity, which is an oligonucleotide having phosphorothioate modification wherein the 5'-position carbon atom of the sugar of at least one nucleoside composing the oligonucleotide forms a structure represented by the following formula I:

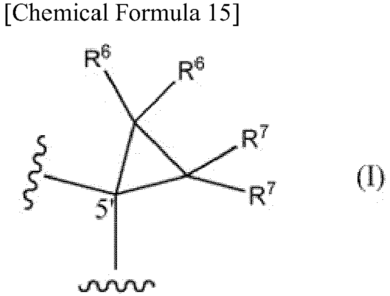

(where the definitions of R⁶ and R⁷ are the same as the definitions of formula I in 1. above), or the following formula I':

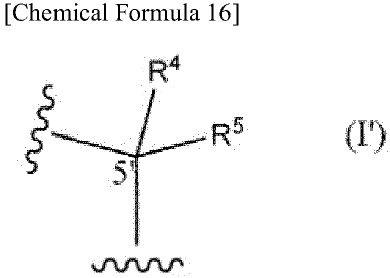

(where the definitions of R⁴ and R⁵ are the same as the definitions of formula I' in 1. above) (this oligonucleotide may also be referred to hereunder as "ASO of the invention"). According to one aspect, R⁶ and R⁷ are both hydrogen atoms. From the viewpoint of further reducing central toxicity, at least one internucleoside bond of the ASO of the invention is preferably a phosphodiester bond.

Therefore, at least one nucleoside composing the ASO of the invention may be represented by the following formula III or III'.

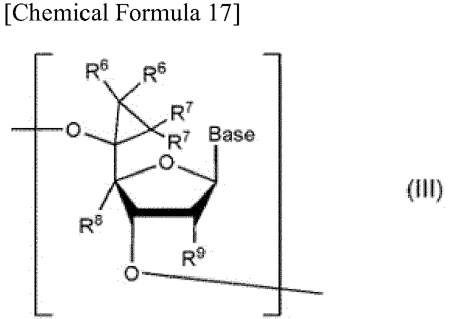

(In the formulas, the definitions of R⁶, R⁷, R⁸, R⁹ and Base are the same definitions as in formula I and formula II of 1. above. According to one aspect, R⁶ and R⁷ are both hydrogen atoms.)

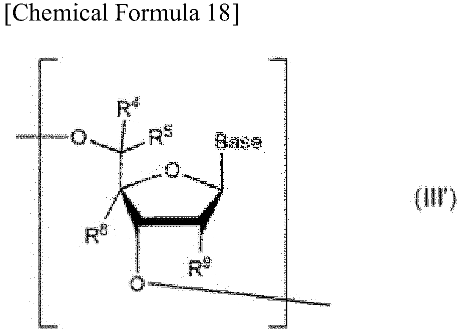

(In the formulas, the definitions of R⁴, R⁵, R⁸, R⁹ and Base are the same definitions as in formula I' and formula II of 1. above.)

The ASO of the invention may be a supression type ASO or an enhancement type ASO. In the case of a supression type ASO, it is preferably a gapmer oligonucleotide, although this is not limitative. In the case of an enhancement type ASO, it is preferably a mixmer oligonucleotide, although this is also not limitative.

A nucleoside having the structure represented by formula I or formula I' may be located at any position of the ASO of the invention, but preferably one or more (e.g.: 1, 2, 3, 4, 5, 6, 7, 8, 9, 10 or more) nucleosides are located in the gap region.

When a phosphodiester bond is present in an internucleoside bond of the ASO of the invention, the phosphodiester bond may be present between any nucleosides of the ASO of the invention, but preferably one or more (e.g.: 1, 2, 3, 4, 5, 6, 7, 8, 9, 10 or more) internucleoside phosphodiester bonds are located in the gap region. When a phosphodiester bond is located in an internucleoside bond of the ASO of the invention, the phosphodiester bond may be adjacent to the 5'-end or 3'-end of a nucleoside with the structure represented by formula I or formula I', or it may be non-adjacent.

In the ASO of the invention, at least one nucleoside composing each region, the 3' wing region and 5' wing region, is preferably a crosslinked nucleoside. The crosslinked nucleoside may be the same type of crosslinked nucleoside as described in 1. above.

The ASO of the invention can suppress or enhance the function of target mRNA, thus regulating expression of a gene. The ASO of the invention can therefore be used as a reagent for regulating expression of a gene (hereunder also referred to as "reagent of the invention"). The term "reagent for regulating expression of a gene" encompasses both the concept of "reagent for suppressing expression of a gene" and "reagent for enhancing expression of a gene".

When the reagent of the invention comprises two or more ASO, or when it includes another reagent type, the reagent may be provided as a reagent kit that comprises each ASO and reagent type in separate reagents. According to the invention, unless otherwise specified, the phrase "expression of a gene" is used to include at least "production of a functional protein encoded by target mRNA" while also including "production of target mRNA". Therefore, suppression of expression of a gene may include not only reduction in the abundance of a functional protein encoded by the gene in cells, but also reduction in the abundance of mRNA transcribed from the gene in cells, resulting from administration of the ASO of the invention. Similarly, enhancement of expression of a gene may include not only increase in the abundance of a functional protein encoded by the gene in cells, but also increase in the abundance of mRNA transcribed from the gene in cells, by administration of the ASO of the invention.

The reagent of the invention may be administered to a subject as the ASO alone, or together with a pharmacologically acceptable carrier. The subject may be cells, tissue or organs of a mammalian animal such as a human, for example. Also provided, therefore, is a method for regulating expression of a gene in a subject that comprises administration of the ASO of the invention to the subject.

In order to promote introduction of the ASO of the invention into the target cells, the reagent of the invention may also include a reagent for nucleic acid introduction. The reagent for nucleic acid introduction may be calcium chloride, a calcium enrichment reagent, atelocollagen, liposomes, nanoparticles, or a cationic lipid such as lipofectin, lipofectamine, DOGS (transfectam), DOPE, DOTAP, DDAB, DHDEAB, HDEAB, polybrene or poly(ethyleneimine) (PEI).

Since the ASO of the invention has reduced central toxicity as mentioned above, it is especially suitable for use as a drug. Further provided, therefore, is an agent for treating for a disease that comprises an ASO of the invention (hereunder also referred to as "therapeutic agent of the invention"). According to the invention, the term "therapeutic" encompasses the concept of alleviating or improving symptoms, and preventing, delaying or stopping progression of a disease or symptoms or manifestation of symptoms. Examples of such conditions include diseases associated with overexpression or excessive accumulation of a protein encoded by target mRNA of the ASO of the invention, or deficiency or reduction in a protein encoded by target mRNA of the ASO of the invention. Examples of such diseases include neuropathies (for example, neurodegenerative diseases such as tauopathy).

The therapeutic agent of the invention may consist of an effective amount of the ASO of the invention alone, or it may be formulated as a pharmaceutical composition together with an optional carrier such as a pharmaceutically acceptable carrier (a formulation will also referred to as "therapeutic agent").

Examples of pharmaceutically acceptable carriers include excipients such as sucrose and starch, binders such as cellulose and methyl cellulose, disintegrators such as starch and carboxymethyl cellulose, lubricants such as magnesium stearate and Aerosil, aromatic agents such as citric acid and menthol, preservatives such as sodium benzoate and sodium bisulfite, stabilizers such as citric acid and sodium citrate, suspending agents such as methyl cellulose, and polyvinylpyrrolidone, dispersing agents such as surfactants, diluents such as water and physiological saline, and base waxes, with no limitation to these.

In order to promote introduction of the ASO of the invention into the target cells, the therapeutic agent of the invention may also comprise a reagent for nucleic acid introduction. The reagent for nucleic acid introduction used in this case may be any of the same ones mentioned above.

The therapeutic agent of the invention may also be a pharmaceutical composition obtained by encapsulating the ASO of the invention in liposomes. Liposomes are microscopic closed vesicles having an inner phase surrounded by one or more lipid bilayers, normally with a water-soluble substance held in the inner phase and a fat-soluble substance in the lipid bilayer. Throughout the present specification, "encapsulation" means either that the ASO of the invention is held in the inner phase of the liposomes, or that it is held in a lipid bilayer. The liposomes to be used for the invention may be a monolayer membrane or multilayer membrane, with particle diameters appropriately selected in the range of 10 to 1000 nm and preferably 50 to 300 nm, for example. In consideration of deliverability to target tissues, the particle diameters are 200 nm or smaller and preferably 100 nm or smaller, for example.

The method selected for encapsulating a water-soluble compound such as an oligonucleotide in liposomes may be any publicly known method without limitations, such as a lipid film method (vortex method), reversed-phase evaporation method, surfactant removal method, freezing-thawing method or remote loading method.

The drug of the invention may be administered perorally or parenterally, to a mammal (e.g.: human, rat, mouse, guinea pig, rabbit, sheep, horse, pig, cow, dog, cat or monkey), but it is preferably administered parenterally. Therefore, also provided is a method for treating a disease, comprising administering an effective amount of the ASO of the invention to a mammal.

Suitable formulations for parenteral administration (such as subcutaneous injection, intramuscular injection, intravenous infusion, local infusion (topical external use or topical application), intracerebroventricular administration, intrathecal administration or intraperitoneal administration) include aqueous and nonaqueous isotonic aseptic injections, which may include antioxidants, buffers, bacteriostatic agents or isotonizing agents. Aqueous and nonaqueous aseptic suspensions may also be used, and may include suspending agents, solubilizing agents, thickeners, stabilizers or antiseptic agents. The formulation may be encapsulated in a unit dosage such as an ampule or vial, or in a multiple-dose container. The active ingredient and the pharmaceutically acceptable carrier may be lyophilized and stored in a form that can be dissolved or suspended in an appropriate aseptic vehicle just prior to use. Another type of formulation suitable for parenteral administration is a spray formulation.

The content of the ASO of the invention in the pharmaceutical composition may be, for example, about 0.1 to 100 wt% of the entire pharmaceutical composition.

The dose of the therapeutic agent of the invention will differ depending on the purpose of administration, the method of administration, the type of target disease, the severity and the condition of the administered individual (such as gender, age and body weight), but in the case of systemic administration to an adult, for example, it is usually preferred to be 0.01 mg/kg to 1000 mg/kg, as a single dose of the ASO of the invention, or for local administration it is preferred to be from 0.001 mg/body to 100 mg/body. The dose is preferably given 1 to 10 times, and more preferably 5 to 10 times.

The therapeutic agent of the invention may also be used in combination with an existing commercial therapeutic agent for a disease. Such a concomitant drug may be formulated together with the drug of the invention and administered as a single formulation, or they may be formulated separately from the drug of the invention and administered either by the same or a different route from the drug of the invention, and either simultaneously or at a different time. The dose of such other concomitant drugs may be doses normally used when the drugs are administered alone, or they may be reduced below the normally used doses.

The invention will now be described in greater detail by Examples, with the understanding that the invention is not limited thereto in any way.

### [EXAMPLES]

### Example 1: Design and synthesis of antisense nucleotides

The following oligonucleotides were designed and synthesized to evaluate the central toxicity of the antisense oligonucleotides. LX-A4285 has a phosphorothioate bond as an internucleoside bond, and has a sequence known to exhibit central toxicity according to WO 2016/127000 A1. In the tables, "5" represents 5-methylcytosine, Ln represents LNA, ^ represents a phosphorothioate bond, and Cp represents DNA with a cyclopropane structure introduced at the 5'-position of the sugar (indicated herein as "5'-cyclopropane DNA").

**[Table 1]**

| Sequence (5' → 3') | Reference sequence | SEQ ID NO: |
|---|---|---|
| A(Ln)^5(Ln)^5(Ln)^a^t^g^a^t^c^t^t^a^g^G(Ln)^5(Ln)^T(Ln) | LX-A4285 | 1 |
| A(Ln)^5(Ln)^5(Ln)A(Cp)^t^g^a^t^c^t^t^a^g^G(Ln)^5(Ln)^T(Ln) | LX-A5105 | 2 |
| A(Ln)^5(Ln)^5(Ln)A(Cp)T(Cp)^g^a^t^c^t^t^a^g^G(Ln)^5(Ln)^T(Ln) | LX-A5106 | 3 |
| A(Ln)^5(Ln)^5(Ln)A(Cp)T(Cp)G(Cp)^a^t^c^t^t^a^g^G(Ln)^5(Ln)^T(Ln) | LX-A5107 | 4 |
| A(Ln)^5(Ln)^5(Ln)A(Cp)T(Cp)G(Cp)A(cp)^t^c^t^t^a^g^G(Ln)^5(Ln)^T(Ln) | LX-A5108 | 5 |

**[Table 2]**

| Sequence (5' → 3') | Reference sequence | SEQ ID NO: |
|---|---|---|
| A(Ln)5(Ln)5(Ln)at^g^a^t^c^t^t^a^g G(Ln)5(Ln)T(Ln) | LX-A4286 | 7 |
| A(Ln)^5(Ln)^5(Ln)A(Cp)T(Cp)^g^a^t^c^t^tA(Cp)G(Cp)^G(Ln)^5(Ln)^T(Ln) | LX-A5113 | 8 |

The oligonucleotides for the invention were synthesized by the methods described in Tetrahedron Letters 22, 1859-1862(1981) and International Patent Publication No. WO2011/052436.

The Locked Nucleic Acid (LNA) represented by formula (a) and the 5'-cyclopropane DNA (5'-CP-DNA) represented by formula (b) were used as sugar-modified nucleosides.

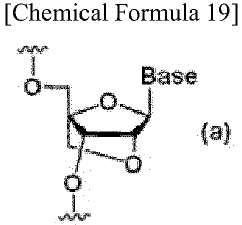

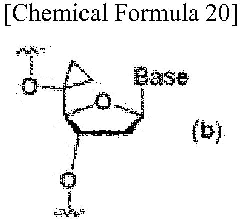

(In these formulas, Base is a 5-methylcytosin-1-yl, thymine-1-yl, adenin-9-yl or guanin-9-yl group.

An oligonucleotide including 5'-cyclopropane DNA (5'-CP-DNA) was synthesized with reference to the method described in International Patent Publication No. WO2020/158910.

An oligonucleotide including Locked Nucleic Acid (LNA) or 5'-cyclopropane DNA (5'-CP-DNA) was synthesized using a nucleic acid automatic synthesizer (Model nS-8, product of GeneDesign, Inc.). Chain length extension was carried out according to standard phosphoroamidite protocol (solid phase carrier: CPG, iodine used for oxidation for phosphoro diester (PO) backbone formation, DDTT (((dimethylamino-methylidene)amino)-3H-1,2,4-dithiazaoline-3-thione) used for sulfidation for formation of phosphorothioate (PS) backbone). The oligonucleotide including Locked Nucleic Acid (LNA) or 5'-cyclopropane DNA (5'-CP) was one in which the terminal 5'-position hydroxyl group was not protected by a DMTr (4,4'-dimethoxytrityl) group, and the 3'-position was supported in the solid phase. Through subsequent treatment of the nucleotide, the portion of interest was cut out from the solid phase carrier, the solvent was distilled off, and the obtained crude product was purified by reverse-phase HPLC to obtain the portion of interest.

The purity and structure of each obtained oligonucleotide was confirmed by LC-MS (product of Waters Co.).

### Example 2: Construction of toxicity evaluation method for antisense nucleotide in mouse central nervous system

Central toxicity of an antisense oligonucleotide was evaluated using BALB/cCrSlc mice (male, 7-week-old, Japan SLC, Inc.). The animals were reared in an open rack in an environment with temperature: 18 to 28°C, humidity: 30 to 80%, illumination time: 12 hr/day, providing all of the animals with free access to feed and water.

The antisense oligonucleotide was administered to the mice by intracerebroventricular administration in order to evaluate central toxicity. Under deep anesthesia produced by intraperitoneal administration of a 3-component mixed anesthetic (5 mg/kg butorphanol tartrate, 4 mg/kg midazolam, 0.3 mg/kg medetomidine hydrochloride) in an amount of 10 mL/kg, the mice were fixed with a stereotaxic device. The cranial bone was exposed, a hole was opened at the administration site using a dental drill (brain coordinates: -0.2 mm posterior to bregma, -1.0 mm right, -2.5 mm deep), and a single dose was intracerebroventricularly administered using a 30G needle, microsyringe and polyethylene tube. The antisense oligonucleotide used was LX-A4285 shown in Table 1.

Behavioral analysis of the mice was conducted by an Irwin test 1 to 24 hours after single dose intracerebroventricular administration of LX-A4285. Scoring was recorded for the evaluation parameters of spontaneous movement, rearing behavior, hyperkinesia, ambulation, grooming, blepharoptosis, vocalization, tremor and convulsion, etc.

The results are shown in Fig. 1. The scores for LX-A4285 were calculated from the divergence from normal scores in the Irwin test, recording the median for each group. LX-A4285 exhibited divergence from a score of near 0 for normal behavior, thus indicating that it produces central toxicity. Thus, a suitable toxicity evaluation method for antisense nucleotides in mouse central nervous system had been established.

### Example 3: Suppression of mRNA expression of antisense nucleotides in in vitro human neuroblasts

The antisense oligonucleotide used in Example 1 was used for evaluation of the MAPT mRNA expression-suppressing effect in human neuroblasts.

The control was without addition of oligonucleotide. For comparison, the oligonucleotide 5(Y)^A(Y)^T(Y)^t^t^a^g^a^a^g^t^c^5(Y)^T(Y)^c (LX-A0070; SEQ ID NO: 6) was used as a negative control (NC). In this sequence, "5" represents 5-methylcytosine and "Y" represents AmNA.

The human neuroblasts used were SH-SY5Y cells (EC94030304-F0, by ECACC Co.). Different antisense oligonucleotides (LX-A0070, LX-A4285, LX-A5106, LX-A5108 and LX-A5113) were each taken up into the SH-SY5Y cells using a commercially available transfection reagent (Lipofectamine 3000 by ThermoFisher Scientific), and the mRNA expression level was measured by qRT-PCR to examine knockdown activity (suppression of mRNA expression). The procedure was as follows.

The SH-SY5Y cells in the logarithmic growth phase were seeded into wells of at 24-well plate at 3.0 × 10⁴/well (including an equal ratio mixed medium of Ham's F12 and Eagle's Minimum Essential Medium (E-MEM), containing 10% fetal bovine serum (FBS)). After 24 hours, each antisense oligonucleotide was added into the wells (antisense oligonucleotide concentration in medium: 1, 3, 10, 30 or 100 nM), and incubation was conducted for at least 24 hours.

After incubation, the cells were collected and an RNA extraction reagent (Nucleo ZOL by MACHEREY-NAGEL) was used for extraction of the total RNA. The total RNA was used as template for reverse transcription and PCR amplification using a nucleic acid amplification reaction reagent (QuantiFast Probe RT-PCR kit by QIAGEN). The nucleic acid amplification reaction was conducted with temperature cycling from 50°C, 10 min → 95°C, 5 min → [(95°C, 10 sec → 60°C, 30 sec) × 40 cycles]. For real-time PCR, the mRNA level of human glyceraldehyde-3-phosphate dehydrogenase (GAPDH) as a housekeeping gene was simultaneously quantified, and the mRNA level of MAPT was evaluated with respect to the mRNA level of GAPDH. The mRNA level for each antisense oligonucleotide is shown as a relative value with 1 as the mRNA level for cells without oligonucleotide addition.

The primer sets used were the following:
(MAPT detection primer set)
TaqMan Gene Expression Assay Hs00902194_m1_4331182 (ThermoFisher Scientific)
(GAPDH detection primer set)
TaqMan Gene Expression Assay Hs02786624_g1_4331182 (ThermoFisher Scientific)

The results are shown in Fig. 2. The antisense oligonucleotides were confirmed to exhibit knockdown activity (suppression of mRNA expression) in a dose-dependent manner, compared to the cells without oligonucleotide addition ("control) and the Negative Control added cells. Thus, an antisense nucleotide having a 5'-modified nucleoside could be evaluated to retain mRNA expression-suppressing activity.

### Example 4: Acute toxicity of antisense nucleotides in mouse central nervous system

Two of the antisense nucleotides listed in Table 1 and Table 2 (LX-A5108 and LX-A5113) were selected as representative antisense nucleotides with low cytotoxicity while retaining knockdown activity (data not shown), and the nucleotides were evaluated for acute toxicity in mouse central nervous system. The evaluation method was carried out according to Example 2. The results are shown in Fig. 3. Based on Fig. 3, reduced central toxicity was confirmed with sequences having 5'-CP and increased phosphodiester bonds, compared to antisense nucleotides with only phosphorothioate bonds.

The results of Examples 3 and 4 confirmed that an antisense nucleotide with a 5'-modified nucleoside has reduced central toxicity while retaining mRNA expression-supressing activity.

### [INDUSTRIAL APPLICABILITY]

An oligonucleotide with reduced central toxicity can be designed by carrying out the present invention. Furthermore, if an oligonucleotide synthesized based on the design results is administered to cells or an individual, it is possible to regulate gene expression with reduced side-effects, so that it can be used as an agent for treating a disease.

The present application claims priority based on Japanese Patent Application No. 2021-078507 (filed on May 6, 2021) and Japanese Patent Application No. 2021-151543 (filed on September 16, 2021), the entirety of the disclosures of which are incorporated herein by reference.

## Claims

1. A method for designing an oligonucleotide having reduced central toxicity, comprising:
(1) a step of replacing the 5'-position carbon atom of the sugar of at least one nucleoside of an oligonucleotide having phosphorothioate modification, with a structure represented by the following formula I: 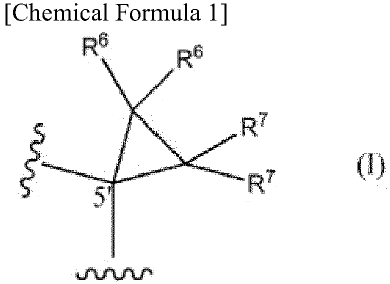 (where R⁶ and R⁷ are each independently a hydrogen atom, halogen atom or methyl group), or the following formula I': 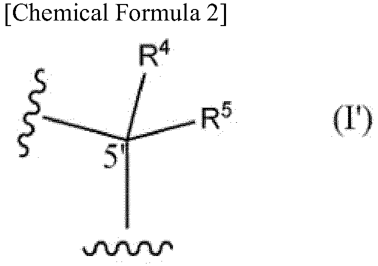 (where R⁴ and R⁵ are each independently a hydrogen atom, methyl group or ethyl group (with the proviso that R⁴ and R⁵ are not both hydrogen atoms)),
and optionally:
(2) a step of replacing at least one phosphorothioate bond of the oligonucleotide with a phosphodiester bond.

2. The method according to claim 1, wherein the oligonucleotide is a gapmer oligonucleotide.

3. The method according to claim 2, wherein at least one nucleoside with the replacement of step (1) is located in the gap region.

4. The method according to claim 2 or 3, wherein at least one phosphorothioate bond with the replacement of step (2) is located in the gap region.

5. The method according to any one of claims 2 to 4, wherein at least one nucleoside composing the 3' wing region and 5' wing region is a crosslinked nucleoside.

6. A method for evaluating the degree of reduction in central toxicity of an oligonucleotide with phosphorothioate modification, comprising the following steps (1) to (3):
(1) a step of preparing an oligonucleotide with phosphorothioate modification,
(2) a step of preparing an oligonucleotide designed by the method according to any one of claims 1 to 5, and
(3) a step of comparing the central toxicity of the oligonucleotide prepared in step (2) with the central toxicity of the oligonucleotide prepared in step (1).

7. An oligonucleotide with a phosphorothioate bond having reduced central toxicity, wherein the 5'-position carbon atom of the sugar of at least one nucleoside of the oligonucleotide forms a structure represented by the following formula I: 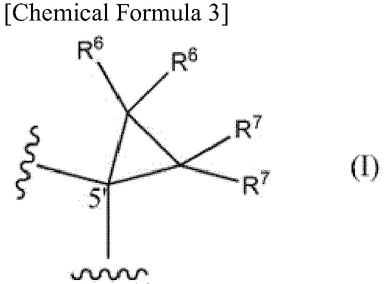 (where R⁶ and R⁷ are each independently a hydrogen atom, halogen atom or methyl group), or the following formula I': 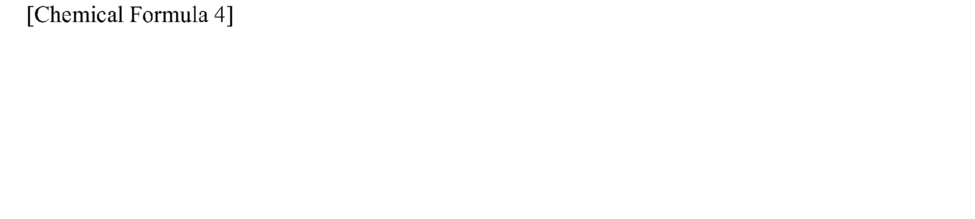 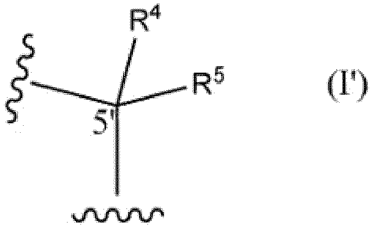 (where R⁴ and R⁵ are each independently a hydrogen atom, methyl group or ethyl group (with the proviso that R⁴ and R⁵ are not both hydrogen atoms)),
and optionally at least one internucleoside bond is a phosphodiester bond.

8. The oligonucleotide according to claim 7, which is a gapmer oligonucleotide.

9. The oligonucleotide according to claim 8, wherein at least one nucleoside having the structure represented by formula I or I' is located in the gap region.

10. The oligonucleotide according to claim 8 or 9, wherein at least one phosphodiester bond is located in the gap region.

11. The oligonucleotide according to any one of claims 8 to 10, wherein at least one nucleoside composing the 3' wing region and 5' wing region is a crosslinked nucleoside.

12. A reagent for regulating expression of a gene, comprising an oligonucleotide according to any one of claims 7 to 11.

13. An agent for treating a disease, comprising an oligonucleotide according to any one of claims 7 to 11.

14. A method for producing an oligonucleotide with low central toxicity, comprising:
(1) a step of designing an oligonucleotide by the method according to any one of claims 1 to 5, and
(2) a step of synthesizing the oligonucleotide designed in step (1).
